Europäisches Patentamt

European Patent Office

Office européen des brevets

(19)

(11) Publication number: **0 013 688**
**B1**

(12) **EUROPEAN PATENT SPECIFICATION**

(45) Date of publication of patent specification: **01.02.84**

(21) Application number: **79103763.3**

(22) Date of filing: **03.10.79**

(51) Int. Cl.³: **C 08 J 3/20, C 08 L 23/06,**
**C 08 K 5/05, C 08 K 5/06,**
**C 08 K 5/12, C 08 K 5/15,**
**C 08 K 11/00**

(54) **A perfumed synthetic resin product and a method of producing said product.**

(30) Priority: **15.12.78 JP 154133/79**

(43) Date of publication of application:
**06.08.80 Bulletin 80/16**

(45) Publication of the grant of the patent:
**01.02.84 Bulletin 84/5**

(84) Designated Contracting States:
**DE FR GB IT**

(56) References cited:
**CH - A - 445 129**
**DE - C - 895 769**
**GB - A - 1 202 796**
**GB - A - 1 390 131**
**GB - A - 1 450 809**
**GB - A - 2 000 516**
**US - A - 3 061 444**
**US - A - 3 140 184**
**US - A - 3 449 266**
**US - A - 3 453 258**
**US - A - 3 472 835**
**US - A - 3 528 819**
**US - A - 3 939 099**

(73) Proprietor: **KYOSHIN CO., LTD.**
**Shinpou Bldg., 3-10-2**
**Honkomagome, Bunkyo-ku, Tokyo (JP)**

(72) Inventor: **Shibanai, Ichiro**
**No. 312 Akasaka Cooperous**
**6-10-6, Akasaka Minato-ku, Tokyo (JP)**
Inventor: **Horikoshi, Kouki**
**8-39-4, Sakuradai**
**Nerima-ku, Tokyo (JP)**
Inventor: **Nakamura, Nobuyuki**
**16-6-1, Naka**
**Kunitachi-city, Tokyo (JP)**

(74) Representative: **Brown, John David et al,**
**FORRESTER & BOEHMERT Widenmayerstrasse**
**4/I**
**D-8000 München 22 (DE)**

Courier Press, Leamington Spa, England.

(56) References cited:
**J. PHARMACEUTICAL SCIENCES 64, (1975), 1596-1599**
**CHEMICAL ABSTRACTS, 84, (1976), 120 071 e**
**CHEMICAL ABSTRACTS, 83, (1975), 146 032 x**

**The file contains technical information submitted after the application was filed and not included in this specification**

A perfumed synthetic resin product and a method of producing said product

The present invention relates to a synthetic resin product which continues emitting a perfume over a long time period and a method of producing the same.

Heretofore, various attempts have been made to develop a synthetic resin product including a perfume. However, perfumery products which are used are in general substances having a high volatility and are generally thermally unstable. Accordingly, it is extremely difficult to obtain a perfumed product by mixing a synthetic resin and the perfumery product(s) and subjecting the mixture to moulding.

It is known that the cyclodextrins may form inclusion compounds with other compounds. This reaction is discussed in the Journal of Pharmaceutical Sciences (1975) Volume 64, Pages 1596 to 1599. DE—C—895769 discloses the use of cyclodextrins as carriers for physiologically active molecules and it is stated that the chemical stability of the guest molecule is thereby increased. Inclusion compounds of cyclodextrins with flavouring agents, medicinal compounds and the like are also described in US—A—3,140,184, GB—A—1,450,809 and the chemical abstracts of the American Chemical Society (1975) Volume 83, 146032X.

A perfumed synthetic resin product is described in GB—A—1,390,131. This product is obtained by coating a polymeric product with a polymeric emulsion containing a volatile oil and allowing the emulsion to dry. GB—A—1,202,796 describes a cosmetic preparation produced by mixing finely divided polyester particles and a perfume.

An object of the present invention, is to provide a perfumed synthetic resin product and a method of producing the same.

According to a first aspect of the present invention there is provided a synthetic resin product produced by moulding a mixture of powdered inclusion compound of cyclodextrin and a perfumery product and a thermoplastic synthetic resin.

According to a second aspect of the present invention there is provided a method of producing a synthetic resin product, which method comprises inclusion compounding a perfumery product with cyclodextrin, dry-powdering the same, mixing the powdered product with a thermoplastic synthetic resin and moulding the same.

Inclusion of the perfumery product by the cyclodextrin results in chemical stabilization thereof, as well as thermal stability, so that it can withstand a relatively high temperature of 260°C to 270°C as well as have mutual resolvability. With a view to the capability of improving its diversity and the mutual resolvability when mixing it into a synthetic resin, a powder of the cyclodextrin/perfumery product inclusion compound is mixed with a thermoplastic synthetic resin material and then the resultant product moulded so as to emit a perfume over a long time period without change in the perfume. For example, lavender oil and cyclodextrin were mixed and the cyclodextrin inclusion compound of lavender oil thus obtained was dried and powdered, and a mixture of the powder and a synthetic resin moulding material was moulded to obtain a synthetic resin product having a lavender perfume. When a citronella oil was used instead of a lavender oil, a perfumed synthetic resin product having an insect repelling effect was obtained.

The following examples further illustrate the present invention.

Example 1

85 parts by weight of $\alpha$-cyclodextrin were added to 15 parts by weight of geraniol and the cyclodextrin inclusion compound thus obtained by stirring and mixing for an hour under a constant temperature of 50°C was powdered by the use of a vacuum dryer or a pulverizing dryer at a dry temperature of 60°C, the particles of the powder being smaller than 150 mesh. The following various moulded goods have been obtained from a mixture of 10 parts by weight of the powder and a 90 parts by weight of polyethylene suitable for moulding.

(1) Using an ejection mould, products such as cups, pencil stands, brushes and toys with a rose perfume have been produced.

(2) Using an inflation processing, a film with a rose perfume for wrapping goods has been produced.

All these products could emit the elegant perfume of rose over a period of half a year without change in the perfume. Especially, portable tissue paper inserted in a bag made of the film of the item (2) above was pleasant in use with the perfume being transferred.

Example 2

The following various moulded products have been obtained from a mixture with 5 parts by weight of the powdered inclusion compound obtained by a method similar to that of Example 1.

(1) Using a fibre obtained by melt spinning, a wig with a rose perfume was manufactured.

(2) By spinning the fibre obtained by melt spinning, a cloth with a rose perfume has been made and goods such as garments, umbrellas, furoshiki (a traditional Japanese wrapping piece of cloth) and curtains have been manufactured. These products could emit the elegant perfume of rose over a half year without change in the perfume.

Example 3

By using dimethyl phthalate instead of the geraniol used in Example 1, a polyethylene film with a sweet perfume and also an insect inhibiting effect has been produced by inflation processing. The perfume and effect was continued nearly a year. However, it is desirable to carry out the manufacture and the powderisation of the inclusion compound in the foregoing embodiment so as to prevent a change in the perfume.

The perfume used include all natural perfumery and synthetic perfumery compounds capable of forming an inclusion compound with cyclodextrin. Furthermore, as the cyclodextrin, instead of or in addition to the $\alpha$-cyclodextrin used in each of the foregoing Examples, $\beta$-cyclodextrin or $\gamma$-cyclodextrin are disclosed in the Japanese Patents Nos 886593 (Publication No. 93/1977, published 5th January 1977) and 914137 (Publication No. 43897/1977, published 2nd November 1977) and Japanese Patent Publication No. 31223/1978 (Published 1st September 1978). Moreover, as a synthetic resin, all kinds of thermoplastic synthetic resin can be used. However, in order to prevent decomposition of the perfumery agent and at the same time to give a sufficiently noticeable perfume, it is desirable to use one which has a relatively low melting point and has a high permeability. In addition, when it is necessary to provide coloured products, the powdered cyclodextrin inclusion compound can be used as a carrier for a colouring agent. This enables the mutual solvability with the synthetic resin to be remarkably improved, so that a more stable colouring is obtainable than when a colouring agent is added separately.

As described in the foregoing, in the synthetic resin product and the method of producing the same according to the present invention, it is possible to keep emitting the stable perfume over a long time period, while economically using the valuable perfumery, and yet the synthetic resin products without the change in perfume can be simply provided, thus yielding an extremely remarkable effect.

## Claims

1. A synthetic resin product produced by moulding a mixture of a powdered inclusion compound of cyclodextrin and a perfumery product and a thermoplastic synthetic resin.

2. A product according to Claim 1, wherein the resin is polyethylene.

3. A method of producing a synthetic resin product, which method comprises inclusion compounding a perfumery product with cyclodextrin, dry-pordering the same, mixing the powdered product with a thermoplastic synthetic resin and moulding the same.

4. A process according to Claim 3, wherein the resin is polyethylene.

## Revendications

1. Produit de résine synthétique obtenu par moulage d'un mélange associant un composé pulvérisé d'inclusion à base de cyclodextrine et d'un produit parfumant, et une résine synthétique thermoplastique.

2. Produit conforme à la revendication 1, où la résine est du polyéthylène.

3. Méthode visant à obtenir un produit de résine synthétique, qui consiste à effectuer une inclusion associant un agent parfumant à la cyclodextrine, à procéder à une pulvérisation à sec, puis à mélanger le produit pulvérisé avec une résine synthétique thermoplastique, et enfin à mouler le produit obtenu.

4. Procédé conforme à la revendication 3, où la résine est du polyéthylène.

## Patentansprüche

1. Kunstharzprodukt, hergestellt durch Formen einer Mischung einer gepulverten Zyclodextrin-Einschlußverbindung une eines Riechstoffproduktes und eines thermoplastischen Kunstharzes.

2. Produkt nach Anspruch 1, worin das Harz Polyethylen ist.

3. Verfahren zum Herstellen eines Kunstharzproduktes, wobei das Verfahren die Herstellung eines in Zyclodextrin eingeschlossenen Riechstoffes, Trocken-Pulverisieren desselben, Mischen des pulverisierten Produktes mit einem thermoplastischen Kunstharz und Formen desselben aufweist.

4. Verfahren nach Anspruch 3, wobei das Harz Polyethylen ist.